# EUROPÄISCHE PATENTSCHRIFT

(11) **EP 3 937 804 B1**
(45) Veröffentlichungstag und Bekanntmachung des Hinweises auf die Patenterteilung: **10.09.2025**
(21) Anmeldenummer: 20719372.3
(22) Anmeldetag: 08.04.2020
(51) Int. Cl.: A61B 17/50, A46B 9/02, A46B 9/12, A46B 13/02, A46B 13/08

(54) **VORRICHTUNG UND PINSELAUFSATZ ZUM ENTFERNEN MILBENARTIGER PARASITEN MITTELS DREHUNG**
DEVICE AND BRUSH ATTACHMENT FOR REMOVING ACARINE PARASITES BY ROTATION
DISPOSITIF ET TÊTE DE BROSSE D'ÉLIMINATION PAR ROTATION DE PARASITES DE TYPE ACARIEN

(30) Priorität: 13.04.2019 DE 102019002743; 03.04.2020 DE 202020101827 U
(43) Veröffentlichungstag der Anmeldung: 19.01.2022
(73) Patentinhaber: malipano GmbH, 95445 Bayreuth (DE); hecht international GmbH, 91595 Burgoberbach (DE)
(72) Erfinder: STEHR, Isabell Kerstin, 95447 Bayreuth (DE); STEHR, Horst, 95447 Bayreuth (DE)
(74) Vertreter: FDST Patentanwälte
(86) Internationale Anmeldenummer: PCT/EP2020/060080
(87) Internationale Veröffentlichungsnummer: WO 2020/212236

(56) Entgegenhaltungen:
- EP-A1- 1 384 418
- EP-A1- 3 607 903
- KR-A- 20090 110 151
- US-A1- 2015 065 927
- US-A1- 2018 098 615

## Beschreibung

Die Erfindung betrifft eine Vorrichtung und einen entsprechenden Pinselaufsatz zum Entfernen eines in die Haut eines Wirts eingedrungenen milbenartigen Parasiten. Die Vorrichtung ist insbesondere dazu vorgesehen und eingerichtet, eine Zecke aus der Haut von Tieren oder Menschen zu entfernen.

Aus der DE 198 276 51 C1 ist eine Vorrichtung zum Entfernen von Zecken mittels aus elastischem Kunststoff gespritzten Greifarmen bekannt, die an einer Stelle zwischen zwei aus einem Gehäuse herausragenden Griffstücken und Greifbacken über einen Stützsteg einstückig miteinander verbunden sind. Durch die Eigenelastizität der nach Art einer Kunststoffpinzette wirkenden Greifarme werden die Greifbacken bei unbetätigter Vorrichtung mit vorgegebener Schließkraft zusammengedrückt.

Aus der EP 1 658 012 B1 ist eine Vorrichtung zum Entfernen von Parasiten oder Zecken aus der Haut von Tieren oder Menschen bekannt, welche eine Spreizeinrichtung zum Spreizen eines Greifwerkzeugs aufweist, das im ungespreizten Zustand einen im Wesentlichen geschlossenen Hohlraum zur Aufnahme des Parasiten oder der Zecke umschließt. Eine in axialer Richtung wirkende Druckvorrichtung dient zur Betätigung des Greifwerkzeugs und einer Drehvorrichtung zur Drehung des Greifwerkzeugs.

In der US 2018/098615 A1 ist ein Keratin-Behandlungskit beschrieben, anhand dessen es eine halbfeste oder feste, nicht-newtonsche kosmetische Zusammensetzung auf eine Oberfläche von keratinischem Gewebe auftragbar ist. Dieses umfasst einen Hybridapplikator, welcher motorisch oszillierend antreibbar ist.

In der KR 2009 0110151 A ist eine Vorrichtung für Nutztiere, insbesondere für Rinder, offenbart, welche eine von einem Motor angetriebene Rotationsbürste aufweist. Anhand diese kann das Nutzvieh gebürstet werden, um Parasiten zu entfernen.

Aus der EP 3 607 903 A1 ist eine Vorrichtung zur sanften Zeckenentfernung durch Rotation bekannt. Diese umfasst einen Elektromotor, anhand dessen ein Pinselaufsatz angetrieben werden kann. Die Haaranordnung des Pinselaufsatzes bildet eine mittig vorgesehene Vertiefung für das Platzieren eines Zeckenkörpers aus.

Der Erfindung liegt die Aufgabe zugrunde, eine besonders geeignete Vorrichtung zum Entfernen eines in die Haut eines Wirts eingedrungenen milbenartigen Parasiten, insbesondere einer Zecke, anzugeben. Insbesondere soll das Entfernen von Zecken aus der Haut eines Wirts ohne Gefährdung dessen Gesundheit ermöglicht werden. Des Weiteren soll ein für die Entfernung der milbenartigen Parasiten, insbesondere der Zecken, besonders geeigneter Pinselaufsatz angegeben werden. Ferner soll hinsichtlich verschiedener Größen von milbenartigen Parasiten, insbesondere Larven, Nymphen und/oder Zecken, ein geeignetes Sortiment mit entsprechenden Pinselaufsätzen bereitgestellt werden.

Diese Aufgabe wird hinsichtlich der Vorrichtung mit den Merkmalen des Anspruchs 1 und bezüglich des Pinselaufsatzes mit den Merkmalen des Anspruchs 14 gelöst.

Die Vorrichtung, die zum Entfernen eines in die Haut eines Wirts eingedrungenen milbenartigen Parasiten, insbesondere einer Zecke, vorgesehen und eingerichtet ist, weist einen in einem Gehäuse aufgenommenen Antrieb und einen nachfolgend auch als Pinselaufsatz bezeichneten Aufsatz mit einer Anzahl von pinsel- oder bürstenartig angeordneten Fasern, nachfolgend als Fasernanordnung bezeichnet, auf. Das Gehäuse ist zweckmäßigerweise zylinderförmig. Es umfasst geeigneter Weise einen Griff in Form einer Griffläche und/oder einer Griffmulde für eine sichere Handhabung der Vorrichtung.

Unter "Aufsatz" wird auch eine, insbesondere hülsenförmige, Halteeinrichtung der pinsel- oder bürstenartig angeordneten Fasern verstanden. Unter "Faser" wird ein filamentartiger, langgestreckter Körper nach Art eines Haares verstanden.

Die Fasernanordnung weist eine, insbesondere konus- oder kegelstumpfartige, vorzugsweise koaxiale, Ausnehmung auf. Mit anderen Worten weist die Fasernanordnung ausgehend von deren Fasernfreienden eine sich axial zur antriebsseitigen Schnittstelle des Pinselaufsatzes hin verjüngende Vertiefung zur Aufnahme eines Parasiten- oder Zeckenkörpers auf. Die Ausnehmung, welche praktisch nach Art einer Haube wirksam ist, ist bevorzugt als (elliptisches) Paraboloid ausgebildet. Eine paraboloidförmige Ausnehmung ist in besonders geeigneter Weise an die typische Form eines Zeckenkörpers angepasst.

Die Fasern der Fasernanordnung des Pinselaufsatzes bildet an deren Faserfreienden entlang des Umfangs der Ausnehmung eine Fasernkranzanordnung mit mindestens einem Fasernkranz, wobei die Kranzbreite der Fasernkranzanordnung größer oder gleich 0,3 mm, vorzugsweise (0,8 ± 0,4) mm, beträgt. Die Kranzbreite ist die halbe Differenz zwischen dem Außenmaß (Außendurchmesser) und dem Innenmaß (Inndurchmesser) der Fasernkranzanordnung an der fasernfreiendseitigen Öffnung (Mündung) der Ausnehmung. Die Kranzbreite kann durch einen einzelnen, radial äußeren Fasernkranz oder durch eine Anzahl von radial äußeren Fasernkränzen bereitgestellt sein. Besonders bevorzugt ist die Fasernkranzanordnung aus zwei (radial äußeren) Fasernkränzen, insbesondere gleicher (axialer) Fasern- bzw. Fasernkranzlänge, gebildet, wobei die Kranzbreite dieser Fasernkranzanordnung vorzugsweise 0,6 mm bis 1,4 mm, besonders bevorzugt 0,7 mm bis 1,2 mm, beträgt.

Der Pinselaufsatz weist antriebsseitig eine Schnittstelle zur Ankopplung an den, insbesondere an einer Stirnseite des Gehäuses zugänglichen, Antrieb auf. Der mit dem Antrieb koppelbare, dies bedeutet mit diesem lösbar verbundene, insbesondere auswechselbare, Pinselaufsatz ist um eine Drehachse rotatorisch antreibbar und weist hierzu antriebsseitig die (mechanische) Schnittstelle auf.

Gemäß einer besonders vorteilhaften Ausgestaltung ist der Pinselaufsatz, also der Aufsatz mit den Fasern ein einteiliges (einstückiges, monolithisches) Spritzgießteil, insbesondere ein Einkomponenten-Spritzgießteil, vorzugsweise aus Kunststoff. Der solcherart hergestellte Pinselaufsatz ist zur Zeckenentfernung aus der Haut eines Wirtes besonders effektiv und darüber hinaus kostengünstig produzierbar.

Der Antrieb weist geeigneter Weise einen Elektromotor und ein Untersetzungsgetriebe auf. Zweckmäßigerweise weist der Antrieb eine Antriebswelle auf, an welche der Pinselaufsatz wellenendseitig drehfest aufsetzbar ist. Die Antriebswelle ist geeigneter Weise an einer Stirnseite des Gehäuses zugänglich und bildet dort die Koppelstelle für den Pinselaufsatz. Die Untersetzung ist vorzugsweise derart bemessen bzw. eingestellt, dass die Drehzahl des Pinselaufsatzes zwischen 30 U/min (Umdrehungen pro Minute) und 150 U/min, insbesondere (70 ± 20) U/min, vorzugsweise (60 ± 10) U/min, oder insbesondere (100 ± 30) U/min, vorzugsweise (120 ± 10) U/min beträgt.

Die Erfindung geht von der Erkenntnis aus, dass einerseits aufgrund des Umstandes, wonach eine Zecke in deren Lebenszyklus häufig die Einstichstelle in der Haut eines Wirtes verlässt und hierzu notwendigerweise den Stech- bzw. Saugapparat (Hypostom) aus der Haut des Wirtes löst, eine zuverlässige Möglichkeit gegeben sein müsste, die Zecke entsprechend zu stimulieren. Andererseits sollte beim Entfernen zuverlässig vermieden werden, dass der aus der Haut des Wirtes austretende Zeckenkörper beschädigt oder abgerissen wird, zumal praktisch lediglich durch eine solche Beschädigung bzw. einen solchen Zeckenabriss die Gefahr der Übertragung von Erregern (Bakterien oder Viren) gegeben ist. Dies gilt unabhängig von der Körpergröße bzw. dem Wachstumsstadium von der Larve (0,3 mm bis 0,8 mm) über die Nymphe (0,8 mm bis 2,0 mm) zur Zecke (ab etwa 2,5 mm).

Die Erfindung geht daher von der Überlegung aus, dass eine Verletzung der Zecke bzw. ein Abreißen (Zeckenabriss), insbesondere deren aus der Haut des Wirtes herausragenden Zeckenkörpers, zuverlässig vermieden ist, wenn im Zuge des Entfernens der Zecke diese an keiner Stelle gedrückt (gequetscht), also keine Druck-, Zug- und/oder Hebelkraft auf die Zecke ausgeübt wird, was durch herkömmliche Greif-, Schlingen- oder Hebelmechanismen praktisch unvermeidbar ist. Dies kann erreicht werden, wenn die Zecke ohne Ausübung von Druck im Zuge des Entfernens aus der Haut des Wirtes lediglich um deren Achse gedreht wird.

Entsprechende Untersuchungen mittels der Vorrichtung haben gezeigt, dass die Zecke - auch im Falle einer Zeckenlarve ab 0,3 mm - bereits nach wenigen Drehbewegungen den Stech- bzw. Saugapparat aus der Haut des Wirtes löst und die Einstichstelle verlässt. Dies wird besonders vorteilhaft unterstützt durch die im Pinselaufsatz vorgesehene und über dem Zeckenkörper platzierte Ausnehmung, so dass die Zecke, während sich diese mit dem Pinselaufsatz dreht, praktisch nicht gedrückt oder gequetscht wird. Hierbei wird erkanntermaßen vermutlich auch der Greifreflex der Zeckenbeine genutzt. Jedenfalls verhängen sich diese mit den Fasern, insbesondere der Fasernkränzen, des Pinselaufsatzes der Vorrichtung, und die Zecke wird mehrfach sanft und ausreichend langsam gedreht.

Nach etwa fünf bis sechs Drehungen, was nur wenigen, insbesondere lediglich bis zu vier, Rotationsintervallen des Pinselaufsatzes mit ca. 10 s (Sekunden) Betätigungsdauer entspricht, ist ein Lösen des Zeckenkörpers bzw. des verankerten Stechapparates und ein Entfernen der praktisch unversehrten Zecke erreicht. Eine Übertragung von Erregern ist auch für den möglichen Fall zuverlässig vermieden, dass möglicherweise die Spitze des Stech- bzw. Saugapparates (Hypostoms) in der Einstichstelle verbleibt, wenn die Zecke die Einstichstelle verlässt. Wesentlich ist, dass mittels der erfindungsgemäßen Vorrichtung, insbesondere mittels des Pinselaufsatzes, ein möglicher "Zeckenabriss" oberhalb des Stech- bzw. Saugapparates der Zecke zuverlässig vermieden ist.

Die Anordnung der Fasern (Fasernanordnung) ist außenseitig vorzugsweise zylindrisch (zylinderförmig). Besonders vorteilhaft ist die Fasernanordnung konzentrisch, wobei die axiale Länge radial innerer Fasern kleiner ist als die axiale Länge radial äußerer Fasern. Hierdurch ist/sind die Ausnehmung bzw. Vertiefung zum Platzieren des aus der Haut des Wirts herausragenden Zeckenkörpers besonders geeignet hergestellt bzw. herstellbar. Die besonders vorteilhafte Kegelstumpfform oder Paraboloidform der Ausnehmung kann in Abhängigkeit der Faserndicke bzw. der Fasernquerschnittsfläche und Längendifferenz der Fasern der radial benachbarten (gedachten) Kreislinien, auf denen die Fasern als Fasernkränze konzentrisch angeordnet sind, in Axialrichtung mehr oder weniger ausgeprägt stufenförmig sein.

Die Fasern sind geeigneter Weise auf zumindest zwei (2), insbesondere maximal zehn (10), vorzugsweise zwei (2) bis sechs (6), weiter bevorzugt drei (3) bis fünf (5), konzentrischen Kreislinien bzw. Fasernkränzen angeordnet. Auf diese Weise sind Pinselaufsätze unterschiedlicher Größe bereitstellbar, beispielsweise ein kleiner Pinselaufsatz mit drei Fasernkränzen und ein mittlerer Pinselaufsatz mit vier Fasernkränzen sowie eine großer Pinselaufsatz mit fünf Fasernkränzen.

In vorteilhafter Ausgestaltung der Fasern weisen diese eine dreieckige Querschnittsform auf. Es hat sich als besonders wirksam herausgestellt, wenn eine Dreieckspitze der Fasern radial nach außen orientiert ist. Zudem ist es besonders vorteilhaft, wenn die der Dreieckspitze gegenüberliegende Dreieckseite kreisbogenförmig (konkav) eingewölbt (gebogen) ist. Hierdurch können die Fasern des jeweiligen Fasernkranzes mit deren kreisbogenförmigen Dreieckseite unter Bildung einer möglichst exakten Kreisform in Umfangsrichtung direkt aneinanderliegend angeordnet werden. Des Weiteren hat sich als effektiv herausgestellt, dass das Faserfreiende der oder jeder Faser in eine Faserspitze ausläuft. Ferner ist es vorteilhaft, wenn die Anordnung der Fasern (Fasernanordnung) am Außenumfang der Fasernfreienden eine radial einwärts gerichtete Verjüngung aufweist.

Der Außendurchmesser der Anordnung der Fasern, also des Pinselaufsatzes im Bereich der Fasernanordnung, ist größer als 1 mm, insbesondere größer oder gleich 2 mm, und kleiner als 20 mm, insbesondere kleiner als 10 mm, vorzugsweise kleiner oder gleich 7 mm, geeigneter Weise (6 ± 3) mm. Die axiale Länge des Aufsatzes mit den Fasern (Pinselaufsatzlänge) ist größer als 10 mm und kleiner als 60 mm, insbesondere zwischen 20 mm und 50 mm, vorzugsweise (34 ± 6) mm.

Um bei der Handhabung der Vorrichtung den Pinselaufsatz gezielt am Parasiten bzw. an der Zecke zu positionieren und dort den Parasiten- bzw. Zeckenkörper sicher in der Ausnehmung (Vertiefung) des Pinselaufsatz, vorzugsweise möglichst zentral, zu platzieren ist eine die Fasernanordnung der umgebende, insbesondere transparente, Zentrierhülse vorgesehen. Diese besteht geeigneter Weise aus Kunststoff. Vorzugsweise ist die Zentrierhülse abnehmbar am Gehäuse aufgesteckt, wobei die Zentrierhülse zweckmäßigerweise mit den Fasernfreienden des Pinselaufsatzes abschließt (fluchtet). Mittels der Zentriehülse ist zudem besonders vorteilhaft eine gezielte Aufsetzkraft des Pinselaufsatzes an dessen Anlagestelle eingestellt und somit eine unerwünscht Druckkraft auf die Zecke im Zuge deren Entfernen aus der Haut des Wirts sicher vermieden.

Vorteilhaft ist ein Sortiment mit einer Anzahl von Pinselaufsätzen unterschiedlicher Größe für die und/oder mit der Vorrichtung bereitgestellt. Dieses Sortiment kann mit beispielsweise drei Pinselaufsätzen verschiedener Größen (groß, mittel, klein) in eine Aufnahmekammer (Pinselkammer) des Gehäuses aufgenommen sein. Der Pinselaufsatz kann grundsätzlich auch manuell zum Entfernen eines in die Haut eines Wirts eingedrungenen milbenartigen Parasiten, insbesondere einer Zecke, verwendet werden.

Besonders vorteilhaft ist auch die Verwendung eines als einteiliges Spritzgießteil hergestellten Pinsels mit einer eine, insbesondere konus- oder kegelstumpfartige oder paraboloidförmige, vorzugsweise koaxiale, Ausnehmung aufweisenden Fasernanordnung zum Entfernen von Zecken aus der Haut eines Wirts mittels einer druckkraftlosen, insbesondere greiferlosen, Drehbewegung des Pinsels.

Nachfolgend werden Ausführungsbeispiele der Erfindung anhand einer Zeichnung näher erläutert. Darin zeigen:
- Fig. 1: in perspektivischer Darstellung eine zum Entfernen einer in die Haut eines Wirts eingedrungenen Zecke vorgesehene und eingerichtete Vorrichtung mit einem Gehäuse und mit einem rotatorisch angetriebenen (antreibbaren) Aufsatz mit einer Anzahl von pinsel- oder bürstenartig angeordneten Fasern (Pinselaufsatz),
- Fig. 2: die Vorrichtung gemäß Fig. 1 in einer Explosionsdarstellung,
- Fig. 3a bis 3c: den Pinselaufsatz einer ersten Variante (groß) in perspektivischer Darstellung, in einer Schnittdarstellung entlang der Linie III-III in Fig. 3a sowie in einer Draufsicht auf eine konzentrische Fasernanordnung des Pinselaufsatzes,
- Fig. 4a bis 4c: den Pinselaufsatz einer zweiten Variante (mittel) in perspektivischer Darstellung, in einer Schnittdarstellung entlang der Linie IV-IV in Fig. 4a sowie in einer Draufsicht auf die wiederum konzentrische Fasernanordnung des Pinselaufsatzes mittlerer Größe,
- Fig. 5a bis 5c: den Pinselaufsatz einer dritten Variante (klein) in perspektivischer Darstellung, in einer Schnittdarstellung entlang der Linie V-V in Fig. 5a sowie in einer Draufsicht auf die ebenfalls konzentrische Fasernanordnung des Pinselaufsatzes kleiner Größe, und
- Fig. 6: in größerem Maßstab eine einzelne Faser des Pinselaufatzes mit Blick auf eine Faserspitze.

Einander entsprechende Teile und Größen sind in allen Figuren mit den gleichen Bezugszeichen versehen.

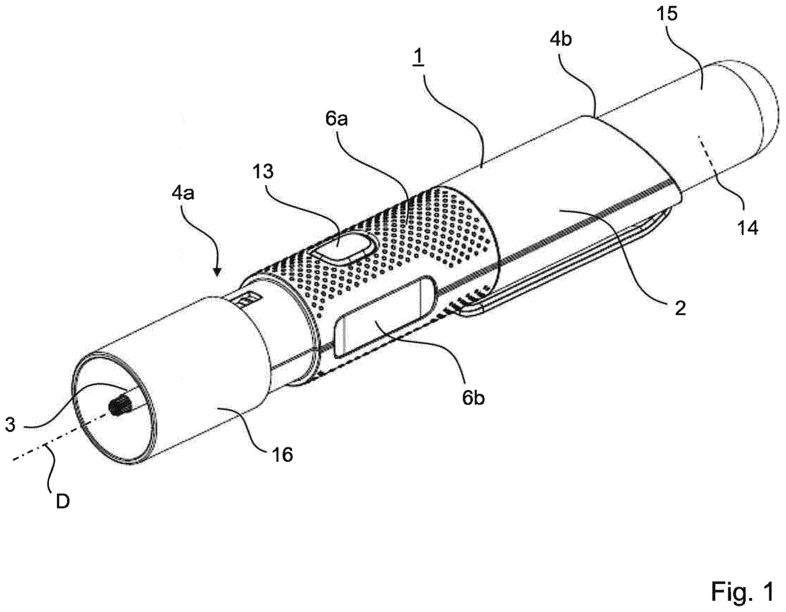

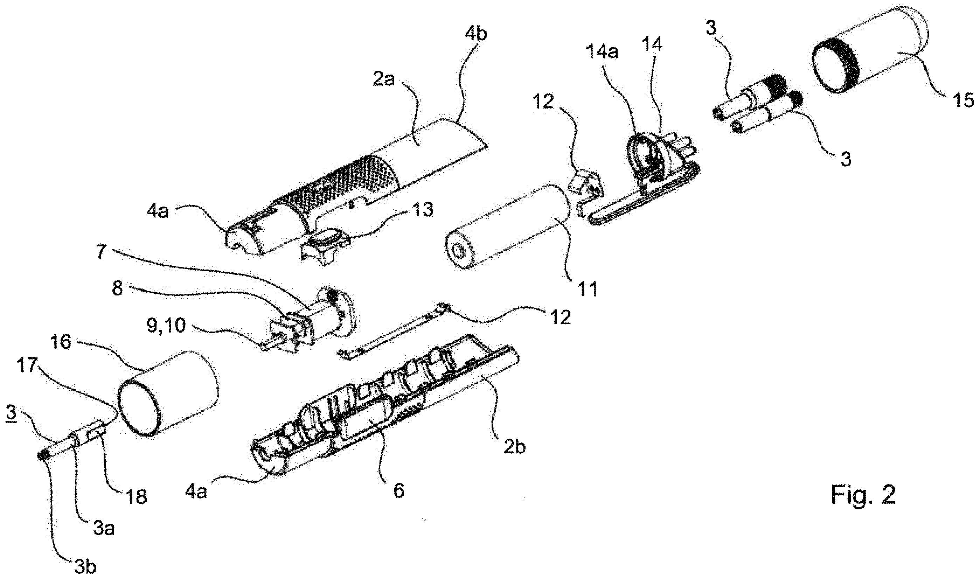

Die in den Figuren 1 und 2 gezeigte Vorrichtung 1 dient zum Entfernen eines in die Haut eines Wirts eingedrungenen milbenartigen Parasiten, insbesondere einer Zecke, und weist ein Gehäuse 2 sowie einen Pinselaufsatz 3 auf, der an einer (vorderen) Gehäusestirnseite 4a an einen in das Gehäuse 2 aufgenommenen Antrieb 5 lösbar gekoppelt ist. Das Gehäuse 2 ist zylinderförmig und umfasst eine Grifffläche mit gegenüber liegenden Griffmulden 6 für eine sichere Handhabung der Vorrichtung 1. Der mit dem Antrieb 5 gekoppelte, auswechselbare Pinselaufsatz 3 ist um eine Drehachse D rotatorisch antreibbar bzw. angetrieben.

Der Antrieb 5 umfasst einen Elektromotor 7 und ein Untersetzungsgetriebe 8 sowie eine Antriebswelle 9, an welche der Pinselaufsatz 3 wellenendseitig drehfest aufsetzbar bzw. aufgesetzt ist. Die Antriebswelle 9 bildet somit an der Gehäusestirnseite 4a eine mechanische Schnittstelle 10 für den Pinselaufsatz 3. Ein in das Gehäuse 2 aufgenommener, insbesondere aufladbarer, Energiespeicher 11 in Form einer Batterie oder eines Akkumulators dient zur Spannungsversorgung bzw. zur Bestromung des Elektromotors 7 und somit zum Betrieb des Antriebs 5. Hierzu sind gehäuseintern Kontaktstücke 12 zur elektrischen Verbindung des Energiespeichers 11 mit dem Elektromotors 8 vorgesehen. Der Antriebs 5 kann mittels eines außen zugänglichen Schalters oder Tasters 13 ein- und ausschaltbar sein. Vorzugsweise ist eine Taster 13 mit Timerfunktionalität vorgesehen. Besonders bevorzugt ist eine Einschaltzeit zwischen 5 s (Sekunden) und 100 s, zweckmäßigerweise 5 s und 25 s eingestellt. Anschließend erfolgt eine automatische Abschaltung des Antriebs 5.

Das Gehäuse 2 ist im Ausführungsbeispiel zweischalig mit einer Oberschale 2a und mit einer Unterschale 2b ausgeführt. An der der vorderen Stirnseite 4a mit der Schnittstelle 11 gegenüberliegenden (hinteren) Gehäusestirnseite 4b des Gehäuses 2 ist dieses mit einer Aufnahmekammer (Pinselkammer) 14 mit einem Kammereinsatz 14a zur Lagerung von Pinselaufsätzen 3 verschiedener Größe vorgesehen. Eine mit dem Gehäuse 2 lösbar verbundene, insbesondere auf dieses aufschraubbare, Kappe (Schraubkappe) 15 deckt die Aufnahmekammer 14 ab. Die Kappe 15 weist ein Außengewinde und das Gehäuse 2 ein korrespondierendes Innengewinde auf. Die Kappe 15 bewirkt mittels des Schraubgewindes eine axiale Druckkraft auf den Kammereinsatz 14a und über diesen die notwendige Kontaktkraft für die Kontaktierung der Kontaktelemente 12 mit dem Energiespeicher 11. Bei abgenommener Kappe 15 ist der Kammereinsatz 14a herausnehmbar, so dass der Energiespeicher 11 und/oder die Kontaktelemente 12 aus dem Gehäuse 2 entnommen werden können.

An der vorderen Stirnseite 4a ist auf das Gehäuse 2 eine vorzugsweise transparente Zentrierhülse 16 aufsetzbar bzw. aufgesetzt. Diese fluchtet freiendseitig mit dem Pinselaufsatz 3.

Mittels der Untersetzung des Antriebs 5 ist eine Drehzahl des Pinselaufsatzes 3 von insbesondere (60 ± 10) U/min oder beispielsweise (120 ± 10) U/min eingestellt. Der Antrieb 5 kann auch für eine stufenweise oder kontinuierliche Drehzahleinstellung eingerichtet sein.

Der Pinselaufsatz 3 ist ein einteiliges, d. h. einstückiges oder monolithisches Spritzgießteil aus Kunststoff. Mit anderen Worten ist der Pinselaufsatz 3 ein Aufsatz 3a mit angeformten Fasern, die pinselartig angeordnet sind, nachfolgend als Fasernanordnung 3b bezeichnet. Die rotatorisch angetriebene Fasernanordnung 3b ist zur Zeckenentfernung aus der Haut eines Wirtes besonders geeignet und vorteilhaft. Der Aufsatz 3a des Pinselaufsatzes 3 bildet die mechanische Schnittstelle 10 für den bzw. zum Antrieb 5. Hierzu weist der Aufsatz 5 eine stirnseitig offene Wellenaufnahme 17, vorzugswiese mit einer Abflachung 18, zur wellenfesten Ankopplung an die Antriebswelle 10 auf. In kinematischer Umkehr kann auch die Antriebswelle 10 wellenendseitig die Aufnahme für einen korrespondierenden Zapfen des Aufsatzes 3b aufweisen. Der Aufsatz 3b ist an der Antriebswelle 10 geeigneter Weise drehfest und lösbar klemmbefestigt.

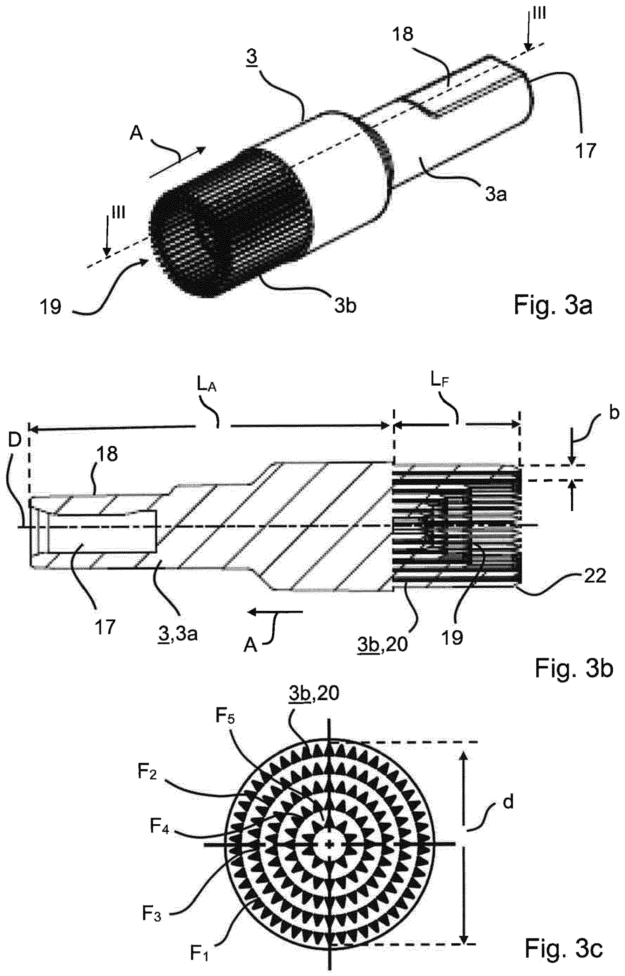

Die Figuren 3a bis 3c zeigen den Pinselaufsatz 3 einer ersten Variante (groß), bevorzugt für eine ausgewachsene Zecke, in perspektivischer Darstellung (Fig. 3a) und in einer Schnittdarstellung (Fig. 3b) entlang der Linie III-III in Fig. 3a sowie in einer (stirnseitigen) Draufsicht auf die bevorzugt konzentrische Fasernanordnung 3a (Fig. 3c).

Die Fasernanordnung 3a ist (außenumfangsseitig) zylindrisch und im Querschnitt kreisförmig. Die Faseranordnung 3a weist eine koaxiale Ausnehmung (Vertiefung) 19 auf. Diese erstreckt sich ausgehend vom Fasernfreiende der Fasernanordnung 3b axial entlang der Drehachse D und verjüngt sich in der gezeigten Axialrichtung A in Richtung zur Drehachse D hin. Die Ausnehmung 19 dient zur Aufnahme eines Parasiten- oder Zeckenkörpers, indem der Pinselaufsatz 3 mit der Ausnehmung (Vertiefung) 19 über den aus der Haut des Wirts herausragenden Zeckenkörper gesetzt wird, so dass dieser nach Art einer Haube überdeckt und in die Ausnehmung 19 aufgenommen ist.

Die Fasernanordnung 3a ist konzentrisch, wobei die axiale Länge innerer Fasern kleiner ist als die axiale Länge radial äußerer Fasern. Mit anderen Worten bilden die Fasern unterschiedlicher Fasernlänge die Ausnehmung 19 zum Platzieren des aus der Haut des Wirts herausragenden Parasiten- oder Zeckenkörpers. Die vorteilhafte Kegelstumpf- oder Paraboloidform der Ausnehmung 19 ist in Abhängigkeit der Faserndicke bzw. der Fasernquerschnittsfläche und der Längendifferenz der Fasern der radial benachbarten (gedachten) Kreislinien, auf denen die Fasern jeweils als Fasernkränze Fₙ (Figuren 3 bis 5) radial beabstandet angeordnet sind, in Axialrichtung A mehr oder weniger ausgeprägt stufenförmig.

Die Ausnehmung 19 hat die Aufgabe, den Zeckenkörper mittels der Fasernanordnung 3b des Pinselaufsatzes 3 haubenartig zu umschließen, ohne den Zeckenkörper zu drücken, zu quetschen oder zu verletzen, sodass sich die Zecke innerhalb der Ausnehmung 19 in der Fasernanordnung 3b des Pinselaufsatzes 3 mit deren Zeckenbeinen verfangenen und beim Einschalten des Gerätes bzw. der Vorrichtung 1 mit der Rotation drehen kann.

Die Fasern des Pinselaufsatzes 3 gemäß den Figuren 3a bis 3c sind auf fünf konzentrischen (gedachten) Kreislinien angeordnet, die jeweils einen Fasernkranz Fₙ bilden. Der Durchmesser d des äußeren Fasernkranzes F₁ der Faseranordnung 3b beträgt im Ausführungsbeispiel d = (7,2 ± 0,2) mm. Der Kreisdurchmesser, auf dem die Fasern aneinander liegen beträgt hier 7 mm. Die nicht näher bezeichneten Durchmesser der sich daran radial einwärts anschließenden Fasernkränze F₂ bis F₅ sind im Ausführungsbeispiel von radial außen nach radial jeweils um jeweils ca. 1,4 mm kleiner. Der Kreisdurchmesser, auf dem diese Fasern aneinander liegen, beträgt zwischen 1,4 mm und 5,6 mm.

Die axiale Länge L_{A} des Aufsatzes beträgt ca. 23 mm. Die axiale Länge L_{F} der Fasern bzw. der Fasernkränze beträgt bei diesem Ausführungsbeispiel für den ersten, äußeren und den zweiten Fasernkranz F₁ und F₂ ca. 8 mm, für den dritten Fasernkranz F₃ ca. 5,0 mm, für den vierten Fasernkranz F₄ ca. 3,5 mm und für den fünften Fasernkranz F₅ ca. 2,5 mm. Die beiden Fasernkränzen F₁ und F₂ bilden eine Fasernkranzanordnung F₁₂, deren (radiale) Kranzbreite b geeigneter Weise (1,0 ± 0,2) mm, beträgt.

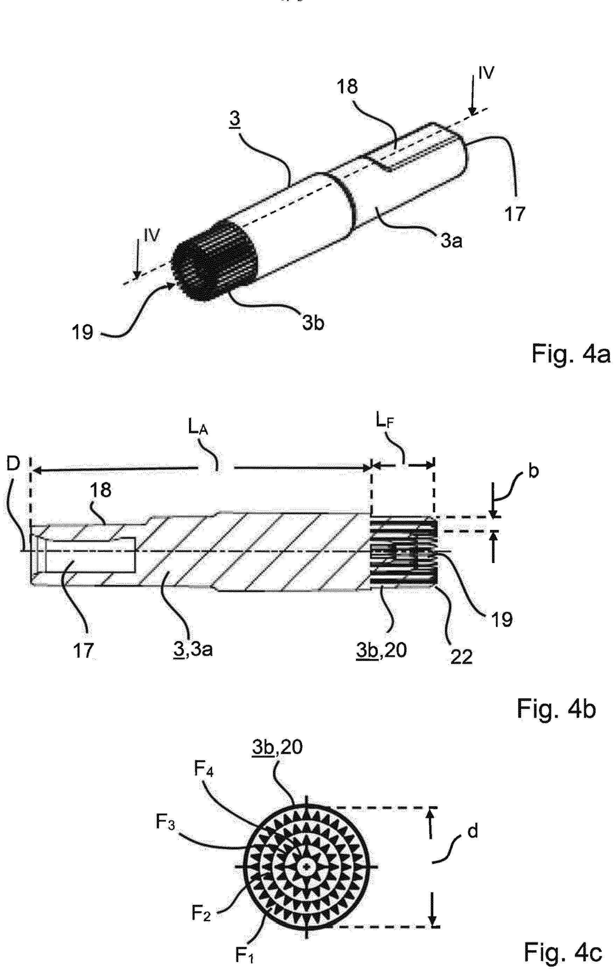

Die Figuren 4a bis 4c zeigen den Pinselaufsatz 3 einer zweiten Variante (mittel), bevorzugt für eine Nymphe, in perspektivischer Darstellung (Fig. 4a) und in einer Schnittdarstellung (Fig. 4b) entlang der Linie IV-IV in Fig. 4a sowie in einer (stirnseitigen) Draufsicht auf die bevorzugt konzentrische Fasernanordnung 3a (Fig. 4c).

Die Fasern des Pinselaufsatzes 3 gemäß den Figuren 4a bis 4b sind auf vier konzentrischen, jeweils wiederum einen Fasernkranz Fₙ bildenden (gedachten) Kreislinien angeordnet. Der Durchmesser d des äußeren Fasernkranzes F₁ der Fasernanordnung 3b beträgt in diesem Ausführungsbeispiel des Pinselaufsatzes 3 mittlerer Größe ca. (4,6 ± 0,2) mm. Die Außendurchmesser der sich daran radial einwärts anschließenden Fasernkränze F₂ bis F₄ sind im Ausführungsbeispiel von radial außen nach radial innen jeweils wiederum um ca. 1,2 mm kleiner. Die beiden äußeren Fasernkränzen F₁ und F₂ bilden wiederum die Fasernkranzanordnung F₁₂.

Die axiale Länge L_{A} des Aufsatzes beträgt ca. 26 mm. Die axiale Länge L_{F} der Fasern bzw. der Fasernkränze Fₙ auf diesen Kreislinien beträgt bei diesem Ausführungsbeispiel für den ersten, äußeren und den zweiten Fasernkranz F₁, F₂ ca. 5 mm, für den dritten Fasernkranz F₃ ca. 3,5 mm und für den vierten Fasernkranz F₄ ca. 1,9 mm. Die (radiale) Kranzbreite b der beiden äußeren Fasernkränzen F₁ und F₂, welche die Fasernkranzanordnung F₁₂ bilden, beträgt geeigneter Weise wiederum (1,0 ± 0,1).

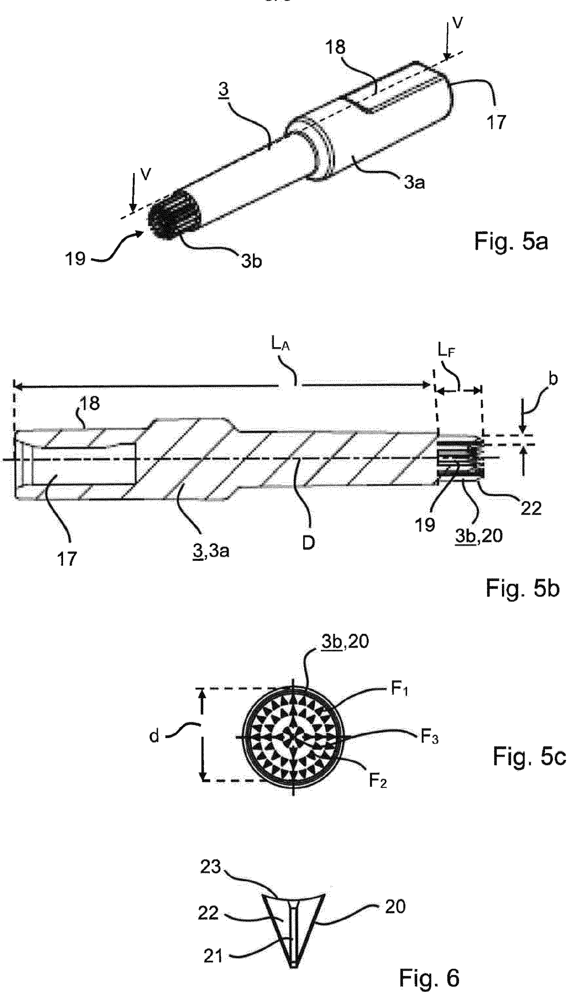

Die Figuren 5a bis 5c zeigen den Pinselaufsatz 3 einer dritten Variante (klein), bevorzugt für eine Larve, in perspektivischer Darstellung (Fig. 5a) und in einer Schnittdarstellung (Fig. 5b) entlang der Linie V-V in Fig. 5a sowie in einer (stirnseitigen) Draufsicht auf die bevorzugt konzentrische Fasernanordnung 3a (Fig. 5c).

Die Fasern des Pinselaufsatzes 3 gemäß den Figuren 5a bis 5b sind auf drei konzentrischen, jeweils wiederum einen Fasernkranz Fₙ bildenden (gedachten) Kreislinien angeordnet. Der Durchmesser d des äußeren Fasernkranzes F₁ der Fasernanordnung 3b beträgt in diesem Ausführungsbeispiel des Pinselaufsatzes 3 kleiner Größe ca. 3,6 mm. Die Außendurchmesser der sich daran radial einwärts anschließenden Fasernkränze F₂ und F₃ ist im Ausführungsbeispiel wiederum um jeweils ca. 1,2 mm kleiner. Die (radiale) Kranzbreite b der beiden äußeren Fasernkränzen F₁ und F₂, welche die Fasernkranzanordnung F₁₂ bilden, beträgt geeigneter Weise wiederum (1,0 ± 0,1) mm.

Die axiale Länge L_{A} des Aufsatzes 3a beträgt bei diesem Ausführungsbeispiel ca. 28 mm. Die axiale Länge L_{F} der Fasern des äußeren Fasernkranzes F₁ beträgt 3 mm. Die axiale Länge der Fasern des hierzu konzentrischen inneren Fasernkranzes F₃ mit alternierend nach außen und innen gerichteten Dreieckspitzen beträgt ca. 2,8 für die Fasern mit nach außen gerichteten Dreieckspitzen und ca. 2,5 mm für die Fasern mit nach innen gerichteten Dreieckspitzen.

Wie in Fig. 6 anhand einer einzelnen Faser 20 des Pinselaufsatzes 3 gezeigt ist, sind die Fasern im Querschnitt dreieckig. Eine der Dreieckspitzen der Fasern 20 ist radial nach außen orientiert. Lediglich bei der Ausführungsform gemäß den Figuren 5a bis 5c sind die Dreieckspitzen der Fasern des inneren Fasernkranzes F₃ in Umfangsrichtung alternierend nach innen und nach außen gerichtet.

Das Faserfreiende der Faser 20 zumindest des äußeren Fasernkranzes F₁ läuft in eine Faserspitze 21 aus. Dabei ist es vorteilhaft, dass die Fasernspitzen 21 eine radial einwärts verlaufende Schräge 22 aufweisen, so dass die Fasernanordnung 3b am Außenumfang der Fasernfreienden - also fasernfreiendseitig - eine radial einwärts gerichtete, von den Schrägen 22 gebildete Verjüngung aufweisen. Hierdurch ist erreicht, dass beim Aufsetzen der Fasernanordnung 3b des Pinselaufsatzes 3 die Fasernfreienden der Fasern bei entsprechender, axialer Druckausübung auf die Haut des Wirts bevorzugt nach innen (radial einwärts) und weit weniger wahrscheinlich nach (radial) außen ausweichen. Hierdurch ist die Wirksamkeit der Vorrichtung 1 beim Entfernen der in die Haut des Wirts eingedrungen Zecke vorteilhaft verbessert.

Die Breite oder Dicke einer (einzelnen) Faser 20 (unterhalb der Faserspitze 21) beträgt vorzugsweise 0,3 mm bis 0,4 mm, beispielsweise an der schmalsten Stelle ca. 0,34 mm und an der breitesten Stelle ca. 0,4 mm. Die der Faserspitze 21 gegenüberliegende Dreieckseite 23 ist kreisbogenförmig einwärts gewölbt. Hierdurch wird in der Anordnung der Fasern 20 zum Fasernkranz Fₙ eine möglichst exakte Kreisform erzielt. Die Fasernkränze Fₙ sind geeigneter Weise zueinander beabstandet, wobei der radiale Abstand benachbarter Fasernkränze Fₙ etwa 0,1 mm bis 0,4 mm, vorzugsweise 0,2 mm bis 0,3 mm, beträgt.

Mittels der Vorrichtung 1 ist bereits nach wenigen Umdrehungen des um die Drehachse D rotatorisch angetriebenen Pinselaufsatzes 3 erreicht, dass die Zecke deren Stechapparat aus der Haut des Wirtes löst und die Einstichstelle verlässt. Hierbei wird durch die koaxiale, sich axial verjüngende Ausnehmung 19 erreicht, dass die Fasern zwischen die Extremitäten der Zecke (Zeckenbeine) gelangen. Dies ermöglicht die gewünschte Drehbewegung der Zecke innerhalb der Einstichstelle und stellt ein zuverlässiges Lösen der Zecke aus der Einstichstelle sicher. Dabei wird die Zecke besonders einfach, zuverlässig und insbesondere praktisch zerstörungsfrei aus der Haut des Wirts entfernt, und die gelöste Zecke befindet sich dann in der Ausnehmung 19 des Pinselaufsatz 3.

Bei der Handhabung der Vorrichtung 1 wird der Pinselaufsatz 3 und insbesondere dessen Fasernanordnung 3b mit der Ausnehmung 19 mittels der transparenten Zentrierhülse 16 gezielt über der Zecke positioniert, um den Zeckenkörper sicher in der Ausnehmung 19 des Pinselaufsatz 3 zu platzieren. Die am Gehäuse 2 aufgesteckte Zentrierhülse 16 fluchtet mit den Faserfreienden des Pinselaufsatzes 3 oder ist gegenüber diesen geringfügig axial zurückversetzt. Die Zentrierhülse 16 kann abnehmbar am Gehäuse 2 aufgesetzt sein. Vorzugsweise ist die Zentrierhülse 16 unverlierbar am Gehäuse 2 angeordnet und zweckmäßigerweise an diesem axial verschiebbar, wobei die Zentrierhülse 16 in der gezeigten vorderen Position und/oder in einer nach hinten gezogener Position verrastet sein kann.

Die zylindrische Zentrierhülse 16 weist einen Durchmesser von 9 mm bis 30 mm, vorzugsweise (20 ± 5) mm auf. Deren Aufgabe ist einerseits die Bereitstellung einer Schutzhülle für den Pinselaufsatz 3, um einen unnötigen Verschleiß oder ein ungewolltes Verbiegen des Pinselaufsatzes 3 zu vermeiden. Andererseits ist sichergestellt, dass ein Benutzer durch das Aufsetzen der Zentrierhülse 16 auf der Haut im Umkreis der mittig des Hülsenkreises zentrierten Zecke einen sicheren Gebrauch durchführen kann. Insbesondere ist mittels der Zentrierhülse 16 ein kontrollierter, möglichst geringer Aufsetzdruck des Pinselaufsatzes 3 auf die Haut des Wirtes eingestellt. Daher sollte die Zentrierhülse 16 vorne (stirnseitig) möglichst exakt mit den Fasernfreienden der Fasern abschließen, sodass die Fasernanordnung 3b und die Zentrierhülse 16 zeitgleich die Haut berühren. Zudem verhindert die Zentrierhülse 16, dass die Zecke nach dem Loslassen herunterfällt.

Die Vorrichtung 1 kann bei deren Benutzung, insbesondere an schwer zugänglichen Körperstellen, ohne bzw. mit axial nach hinten geschobener Zentrierhülse 16 angewendet werden. Nach der Benutzung sollte der Pinselaufsatz 3 gereinigt, desinfiziert oder bei Notwenigkeit ausgetauscht werden.

Zusammenfassend betrifft die Erfindung eine Vorrichtung 1 zum Entfernen eines in die Haut eines Wirts eingedrungenen milbenartigen Parasiten, aufweisend einen in einem Gehäuse 2 aufgenommenen Antrieb 5 und einen um eine Drehachse drehbaren Aufsatz 3, 3a mit einer pinselartigen Fasernanordnung 3b, die eine Ausnehmung 19 aufweist, wobei eine von den Fasern an deren Faserfreienden entlang des Umfangs der Ausnehmung 19 gebildete Fasernkranzanordnung F₁₂ eine Kranzbreite b zwischen 0,3 mm und kleiner als 1,5 mm, insbesondere (0,8 ± 0,4) mm, aufweist. In besonders vorteilhafter Ausgestaltung ist der Aufsatz 3a mit den Fasern bzw. mit der Fasernanordnung 3b - also der Pinselaufsatz 3 - ein einteiliges Spritzgießteil, insbesondere aus Kunststoff.

Die beanspruchte Erfindung ist nicht auf die vorstehend beschriebenen Ausführungsbeispiele beschränkt. Vielmehr können auch andere Varianten der Erfindung von dem Fachmann hieraus im Rahmen der offenbarten Ansprüche abgeleitet werden, ohne den Gegenstand der beanspruchten Erfindung zu verlassen. So kann der Pinselaufsatz auch - quasi als Pinsel - manuell zum Entfernen eines in die Haut eines Wirts eingedrungenen milbenartigen Parasiten, insbesondere einer Zecke, verwendet werden.

### Bezugszeichenliste

- 1: Vorrichtung
- 2: Gehäuse
- 2a: Oberschale
- 2b: Unterschale
- 3: Pinselaufsatz
- 3a: Aufsatz
- 3b: Faseranordnung
- 4a: (vordere) Gehäusestirnseite
- 4b: (hintere) Gehäusestirnseite
- 5: Antrieb
- 6a: Grifffläche
- 6b: Griffmulde
- 7: Elektromotor
- 8: Untersetzungsgetriebe
- 9: Antriebswelle
- 10: Schnittstelle
- 11: Energiespeicher
- 12: Kontaktelement/-stück
- 13: Schalter/Taster
- 14: Aufnahmekammer
- 14a: Kammereinsatz
- 15: Kappe
- 16: Zentrierhülse
- 17: Wellenaufnahme
- 18: Abflachung
- 19: Ausnehmung/Vertiefung
- 20: Faser
- 21: Faserspitze
- 22: Schräge/Verjüngung

- A: Axialrichtung
- Fₙ: Fasernkranz
- D: Drehachse

- b: Kranzbreite
- d: Durchmesser
- L_{A}: Aufsatzlänge
- L_{F}: Fasernlänge

## Patentansprüche

1. Vorrichtung (1) zum Entfernen eines in die Haut eines Wirts eingedrungenen milbenartigen Parasiten, insbesondere einer Zecke, aufweisend einen in einem Gehäuse (2) aufgenommenen, insbesondere elektromotorischen, Antrieb (5), und einen antriebsseitig eine Schnittstelle (10) aufweisenden, um eine Drehachse (D) rotatorisch antreibbaren Aufsatz (3, 3a) mit einer Anzahl von pinselartig angeordneten Fasern (20) als Fasernanordnung (3b),
- wobei die Fasernanordnung (3b) eine, insbesondere konus- oder kegelstumpfartige oder paraboloidförmige, vorzugsweise koaxiale, Ausnehmung (19) aufweist,
- wobei eine von den Fasern (20) an deren Faserfreienden entlang des Umfangs der Ausnehmung (19) gebildete Fasernkranzanordnung mit mindestens einem Fasernkranz (Fₙ) eine Kranzbreite (b) größer oder gleich 0,3 mm, vorzugsweise (0,8 ± 0,4) mm, aufweist, **dadurch gekennzeichnet dass** die Kranzbreite (b) kleiner als 1,5 mm, insbesondere kleiner oder gleich 1,4 mm, ist.

2. Vorrichtung (1) nach Anspruch 1,
**dadurch gekennzeichnet,**
**dass** die Fasernkranzanordnung aus zwei Fasernkränzen (Fₙ) gebildet ist, deren Kranzbreite (b) zwischen 0,4 mm und 1,2 mm, vorzugsweise (1,0 ± 0,2) mm, beträgt.

3. Vorrichtung (1) nach Anspruch 1 oder 2,
**dadurch gekennzeichnet,**
**dass** der Aufsatz (3, 3a) mit der Fasernanordnung (3b) ein einteiliges Spritzgießteil, insbesondere aus Kunststoff, ist.

4. Vorrichtung (1) nach einem der Ansprüche 1 bis 3,
**dadurch gekennzeichnet,**
**dass** die Fasernanordnung (3b) konzentrisch ist, wobei die axiale Länge der inneren Fasern (20) kleiner ist als die axiale Länge der radial äußeren Fasern (20).

5. Vorrichtung (1) nach einem der Ansprüche 1 bis 4,
**dadurch gekennzeichnet,**
**dass** die Fasern (20) auf zumindest zwei, insbesondere maximal zehn, vorzugsweise zwei bis sechs, konzentrischen Fasernkränzen (Fₙ) angeordnet sind.

6. Vorrichtung (1) nach einem der Ansprüche 1 bis 5,
**dadurch gekennzeichnet,**
- **dass** die Fasern (20) einen dreieckigen Faserquerschnitt, insbesondere mit radial nach außen orientierter Dreieckspitze der Fasern (20), aufweisen.

7. Vorrichtung (1) nach Anspruch 6,
**dadurch gekennzeichnet,**
**dass** die Fasernanordnung (3b) am Außenumfang (5) der Faserfreienden eine radial einwärts gerichtete Verjüngung (22) aufweist.

8. Vorrichtung (1) nach einem der Ansprüche 1 bis 7,
**dadurch gekennzeichnet,**
**dass** der Außendurchmesser (d) der Fasernanordnung (3b) größer als 1 mm, insbesondere größer oder gleich 2 mm, und kleiner als 20 mm, insbesondere kleiner als 10 mm, vorzugsweise kleiner oder gleich 7 mm, ist.

9. Vorrichtung (1) nach einem der Ansprüche 1 bis 8,
**dadurch gekennzeichnet,**
- **dass** die Länge (L_{A}) des Aufsatzes (3, 3a) mit der Fasernanordnung (3b) größer als 10 mm und kleiner als 60 mm, insbesondere zwischen 20 mm und 50 mm, vorzugsweise (34 ± 6) mm, ist, und/oder
- **dass** die Länge (L_{F}) der Fasernanordnung (3b) größer als 1,5 mm und kleiner als 30 mm, vorzugsweise (6 ± 4) mm, ist.

10. Vorrichtung (1) nach einem der Ansprüche 1 bis 9,
**gekennzeichnet**
**durch** eine die Fasernanordnung (3b) umgebende, insbesondere transparente, Zentrierhülse (16).

11. Vorrichtung (1) nach einem der Ansprüche 1 bis 10,
**dadurch gekennzeichnet,**
**dass** der Antrieb (5) eine die mechanische Schnittstelle (10) bildende Antriebswelle (9) aufweist, an welche der Aufsatz (3, 3a) mit der Fasernanordnung (3b) wellenendseitig drehfest aufsetzbar ist.

12. Vorrichtung (1) nach einem der Ansprüche 1 bis 11,
**dadurch gekennzeichnet,**
**dass** der Antrieb (5) einen Elektromotor (7) und ein Untersetzungsgetriebe (8) aufweist, wobei die Untersetzung derart eingestellt ist, dass die Drehzahl des Aufsatzes (3, 3a) mit der Fasernanordnung (3b) zwischen 30 U/min und 150 U/min, insbesondere (60 ± 10) U/min oder (120 ± 10) U/min, beträgt.

13. Vorrichtung (1) nach einem der Ansprüche 1 bis 12,
**dadurch gekennzeichnet,**
**dass** das Faserfreiende der oder jeder Faser (20) in eine Faserspitze (21) ausläuft.

14. Pinselaufsatz (3) mit einer Anzahl von konzentrisch angeordneten Fasern (20) oder Fasernkränzen als Faseranordnung (3b) zum Entfernen eines in die Haut eines Wirts eingedrungenen milbenartigen Parasiten für eine Vorrichtung (1) nach einem der Ansprüche 1 bis 13,
- wobei die Fasernanordnung (3b) eine, insbesondere konus- oder kegelstumpfartige oder paraboloidförmige, vorzugsweise koaxiale, Ausnehmung (19) aufweist,
- wobei eine von den Fasern (20) an deren Faserfreienden entlang des Umfangs der Ausnehmung (19) gebildete Fasernkranzanordnung mit mindestens einem Fasernkranz (Fₙ) eine Kranzbreite (b) größer oder gleich 0,3 mm, vorzugsweise (0,8 ± 0,4) mm, aufweist, **dadurch gekennzeichnet dass** die Kranzbreite (b) kleiner als 1,5 mm, insbesondere kleiner oder gleich 1,4 mm, ist.

## Claims

1. Device (1) for removing an acarine parasite that has penetrated into a host's skin, in particular a tick, the device having a drive (5), in particular an electric motor drive, accommodated in a housing (2), and an attachment (3, 3a) that has an interface (10) on the drive side, is drivable in rotation about an axis of rotation (D) and has a number of fibres (20) arranged in the manner of a brush as fibre arrangement (3b),
- wherein the fibre arrangement (3b) has an in particular conical or frustoconical or paraboloid-shaped, preferably coaxial, recess (19),
- wherein a fibre ring arrangement formed by the fibres (20) at the free ends of the fibres along the circumference of the recess (19) and having at least one fibre ring (Fₙ) has a ring width (b) of greater than or equal to 0.3 mm, preferably (0.8 ± 0.4) mm, **characterized in that**
the ring width (b) is less than 1.5 mm, in particular less than or equal to 1.4 mm.

2. Device (1) according to Claim 1,
**characterized in that**
the fibre ring arrangement is formed from two fibre rings (Fₙ), the ring width (b) of which is between 0.4 mm and 1.2 mm, preferably (1.0 ± 0.2) mm.

3. Device (1) according to Claim 1 or 2,
**characterized in that**
the attachment (3, 3a) together with the fibre arrangement (3b) is a one-piece injection moulding, in particular made of plastic.

4. Device (1) according to any one of Claims 1 to 3,
**characterized in that**
the fibre arrangement (3b) is concentric, with the axial length of the inner fibres (20) being shorter than the axial length of the radially outer fibres (20).

5. Device (1) according to any one of Claims 1 to 4,
**characterized in that**
the fibres (20) are arranged in at least two, in particular not more than ten, preferably two to six, concentric fibre rings (Fₙ).

6. Device (1) according to any one of Claims 1 to 5,
**characterized in that**
- the fibres (20) have a triangular fibre cross section, in particular with a triangular tip of the fibres (20) oriented radially outwards.

7. Device (1) according to Claim 6,
**characterized in that**
the fibre arrangement (3b) has a radially inwardly directed taper (22) at the outer circumference (5) of the free ends of the fibres.

8. Device (1) according to any one of Claims 1 to 7,
**characterized in that**
the outer diameter (d) of the fibre arrangement (3b) is greater than 1 mm, in particular greater than or equal to 2 mm, and less than 20 mm, in particular less than 10 mm, preferably less than or equal to 7 mm.

9. Device (1) according to any one of Claims 1 to 8,
**characterized in that**
- the length (L_{A}) of the attachment (3, 3a) with the fibre arrangement (3b) is greater than 10 mm and less than 60 mm, in particular between 20 mm and 50 mm, preferably (34 ± 6) mm, and/or
- the length (L_{F}) of the fibre arrangement (3b) is greater than 1.5 mm and less than 30 mm, preferably (6 ± 4) mm.

10. Device (1) according to any one of Claims 1 to 9,
**characterized by**
a centring sleeve (16), in particular a transparent centring sleeve, that surrounds the fibre arrangement (3b).

11. Device (1) according to any one of Claims 1 to 10,
**characterized in that**
the drive (5) has a drive shaft (9) which forms the mechanical interface (10) and onto which the attachment (3, 3a) with the fibre arrangement (3b) can be mounted for conjoint rotation on the shaft end.

12. Device (1) according to any one of Claims 1 to 11,
**characterized in that**
the drive (5) has an electric motor (7) and a reduction gear (8), the reduction gear being set such that the speed of the attachment (3, 3a) with the fibre arrangement (3b) is between 30 rpm and 150 rpm, in particular (60 ± 10) rpm or (120 ± 10) rpm.

13. Device (1) according to any one of Claims 1 to 12,
**characterized in that**
the free end of the fibre of each or every fibre (20) runs out into a fibre tip (21).

14. Brush attachment (3) having a number of concentrically arranged fibres (20) or fibre rings as a fibre arrangement (3b) for removing an acarine parasite that has penetrated into a host's skin, for a device (1) according to any one of Claims 1 to 13,
- wherein the fibre arrangement (3b) has an in particular conical or frustoconical or paraboloid-shaped, preferably coaxial, recess (19),
- wherein a fibre ring arrangement formed by the fibres (20) at the free ends of the fibres along the circumference of the recess (19) and having at least one fibre ring (Fₙ) has a ring width (b) of greater than or equal to 0.3 mm, preferably (0.8 ± 0.4) mm, **characterized in that**
the ring width (b) is less than 1.5 mm, in particular less than or equal to 1.4 mm.

## Revendications

1. Dispositif (1) pour enlever un parasite de type acarien, notamment une tique, qui a pénétré dans la peau d'un hôte, présentant un entraînement (5), notamment électromoteur, logé dans un boîtier (2), et un embout (3, 3a) présentant du côté de l'entraînement une interface (10) et pouvant être entraîné en rotation autour d'un axe de rotation (D), avec un nombre de fibres (20) agencées à la manière d'un pinceau, en tant qu'agencement de fibres (3b),
- l'agencement de fibres (3b) présentant un évidement (19), notamment de forme conique ou tronconique ou parabolique, de préférence coaxial,
- un agencement de couronne de fibres formé par les fibres (20) à leurs extrémités libres de fibres le long de la périphérie de l'évidement (19) avec au moins une couronne de fibres (Fₙ) présentant une largeur de couronne (b) supérieure ou égale à 0,3 mm, de préférence (0,8 ± 0,4) mm, **caractérisé en ce que**
la largeur de couronne (b) est inférieure à 1,5 mm, notamment inférieure ou égale à 1,4 mm.

2. Dispositif (1) selon la revendication 1,
**caractérisé**
**en ce que** l'agencement de couronnes de fibres est formé de deux couronnes de fibres (Fₙ) dont la largeur de couronne (b) est comprise entre 0,4 mm et 1,2 mm, de préférence (1,0 ± 0,2) mm.

3. Dispositif (1) selon la revendication 1 ou 2,
**caractérisé**
**en ce que** l'embout (3, 3a) avec l'agencement de fibres (3b) est une pièce moulée par injection d'une seule pièce, notamment en matière plastique.

4. Dispositif (1) selon l'une quelconque des revendications 1 à 3,
**caractérisé**
**en ce que** l'agencement de fibres (3b) est concentrique, la longueur axiale des fibres intérieures (20) étant inférieure à la longueur axiale des fibres radialement extérieures (20).

5. Dispositif (1) selon l'une quelconque des revendications 1 à 4,
**caractérisé**
**en ce que** les fibres (20) sont agencées sur au moins deux, notamment au maximum dix, de préférence deux à six, couronnes de fibres concentriques (Fₙ).

6. Dispositif (1) selon l'une quelconque des revendications 1 à 5,
**caractérisé**
- **en ce que** les fibres (20) présentent une section transversale de fibre triangulaire, notamment avec une pointe de triangle des fibres (20) orientée radialement vers l'extérieur.

7. Dispositif (1) selon la revendication 6,
**caractérisé**
**en ce que** l'agencement de fibres (3b) présente, sur la périphérie extérieure (5) des extrémités libres des fibres, un rétrécissement (22) dirigé radialement vers l'intérieur.

8. Dispositif (1) selon l'une quelconque des revendications 1 à 7,
**caractérisé**
**en ce que** le diamètre extérieur (d) de l'agencement de fibres (3b) est supérieur à 1 mm, notamment supérieur ou égal à 2 mm, et inférieur à 20 mm, notamment inférieur à 10 mm, de préférence inférieur ou égal à 7 mm.

9. Dispositif (1) selon l'une quelconque des revendications 1 à 8,
**caractérisé**
- **en ce que** la longueur (L_{A}) de l'embout (3, 3a) avec l'agencement de fibres (3b) est supérieure à 10 mm et inférieure à 60 mm, notamment entre 20 mm et 50 mm, de préférence (34 ± 6) mm, et/ou
- **en ce que** la longueur (L_{F}) de l'agencement de fibres (3b) est supérieure à 1,5 mm et inférieure à 30 mm, de préférence (6 ± 4) mm.

10. Dispositif (1) selon l'une quelconque des revendications 1 à 9,
**caractérisé**
**par** un manchon de centrage (16), notamment transparent, entourant l'agencement de fibres (3b).

11. Dispositif (1) selon l'une quelconque des revendications 1 à 10,
**caractérisé**
**en ce que** l'entraînement (5) présente un arbre d'entraînement (9) formant l'interface mécanique (10), sur lequel l'embout (3, 3a) avec l'agencement de fibres (3b) peut être monté solidairement en rotation du côté de l'extrémité de l'arbre.

12. Dispositif (1) selon l'une quelconque des revendications 1 à 11,
**caractérisé**
**en ce que** l'entraînement (5) présente un moteur électrique (7) et un réducteur (8), le réducteur étant réglé de telle sorte que la vitesse de rotation de l'embout (3, 3a) avec l'agencement de fibres (3b) est comprise entre 30 tr/min et 150 tr/min, notamment (60 ± 10) tr/min ou (120 ± 10) tr/min.

13. Dispositif (1) selon l'une quelconque des revendications 1 à 12,
**caractérisé**
**en ce que** l'extrémité libre de fibre de la ou de chaque fibre (20) se termine par une pointe de fibre (21).

14. Embout de pinceau (3) avec un nombre de fibres (20) ou de couronnes de fibres agencées de manière concentrique en tant qu'agencement de fibres (3b) pour éliminer un parasite de type acarien qui a pénétré dans la peau d'un hôte, pour un dispositif (1) selon l'une quelconque des revendications 1 à 13,
- l'agencement de fibres (3b) présentant un évidement (19), notamment de forme conique ou tronconique ou parabolique, de préférence coaxial,
- un agencement de couronne de fibres formé par les fibres (20) à leurs extrémités libres de fibres le long de la périphérie de l'évidement (19) avec au moins une couronne de fibres (Fₙ) présentant une largeur de couronne (b) supérieure ou égale à 0,3 mm, de préférence (0,8 ± 0,4) mm, **caractérisé en ce que**
la largeur de couronne (b) est inférieure à 1,5 mm, notamment inférieure ou égale à 1,4 mm.
